# Europäisches Patentamt

# European Patent Office

# Office européen des brevets

(19)

(11) Veröffentlichungsnummer: **0 023 260**
**B1**

(12) **EUROPÄISCHE PATENTSCHRIFT**

(45) Veröffentlichungstag der Patentschrift:
**12.01.83**

(51) Int. Cl.³: **C 07 D 235/26**

(21) Anmeldenummer: **80103381.2**

(22) Anmeldetag: **18.06.80**

(54) **5-Amino-benzimidazol-2-on-Hydrat, Verfahren zu seiner Herstellung und seine Verwendung als Farbenvorprodukt.**

(30) Priorität: **25.06.79 DE 2925541**

(43) Veröffentlichungstag der Anmeldung:
**04.02.81 Patentblatt 81/5**

(45) Bekanntmachung des Hinweises auf die Patenterteilung:
**12.01.83 Patentblatt 83/2**

(84) Benannte Vertragsstaaten:
**CH DE FR GB LI**

(56) Entgegenhaltungen:
**DE-A-2 606 506**
**Chemical Abstracts Band 72, Nr. 7, 16. Februar 1970**
**Columbus, Ohio, USA S.N. KOLODYAZHNAYA et al.**
**«Benzimidazole derivatives. XXI. Bromination of N-alkyl**
**derivatives of 2-aminobenzimidazole and 2-iminobenz-**
**imidazoline» Seite 326, Spalte 1, Abstract Nr. 31697y**
**Chemical Abstracts Band 52, Nr. 16, 25. August 1958,**
**Columbus, Ohio, USA R.L. CLARK et al. «Synthesis of**
**some substituted benzimidazolinones» Spalte 13712 e-f**
**MONATSHEFTE FÜR CHEMIE, Band 107, Nr. 6, 1976 H.**
**SCHINDLBAUER et al. «Zur direkten Nitrierung des**
**Benzimidazolons und der Reduktion einiger dieser Ni-**
**trierungsprodukte» Seiten 1307 bis 1310**

(73) Patentinhaber: **HOECHST AKTIENGESELLSCHAFT,**
**Postfach 80 03 20, D-6230 Frankfurt/Main 80 (DE)**

(72) Erfinder: **Arndt, Otto, Dr., Frankfurter Strasse 38,**
**D-6238 Hofheim am Taunus (DE)**
Erfinder: **Lemberg, Rainer, Kirschblütenstrasse 101,**
**D-6200 Wiesbaden (DE)**

5-Amino-benzimidazol-2-on-Hydrat, Verfahren zu seiner Herstellung und seine Verwendung als Farbenvorprodukt

5-Amino-benzimidazolon-(2) kann als Diazokomponente und als N-Acetoacetyl-Verbindung als Kupplungskomponente für Azoverbindungen, insbesondere Azopigmente, Verwendung finden.

Aus Chem. Ber. 17 (1884), 2631, ist es bekannt, 5-Aminobenzimidazolon-(2) durch Ringschlussreaktion aus N-(2,4-Diamino-phenyl)-äthylurethan unter Alkoholabspaltung herzustellen. Abgesehen davon, dass dieses Ausgangsmaterial schwer zugänglich ist, fällt bei diesem Verfahren das 5-Amino-benzimidazolon-(2), in ungenügender Ausbeute und Reinheit an.

Aus Monatshefte für Chemie 107 (1976), 1307–1310, ist es bekannt, 5-Nitro-benzimidazolon-(2) in methanolischer Lösung mit Hydrazin-Hydrat und Raney-Nickel zu reduzieren. Es wird ausdrücklich erwähnt, dass sich von den möglichen Reduktionsmethoden nur die mit Hydrazinhydrat und Raney-Nickel als günstig erwiesen haben.

Dieses Verfahren ist jedoch wegen der Verwendung des teuren Reduktionsmittels Hydrazin und des organischen Lösemittels, das regeneriert werden muss, ausserordentlich aufwendig. Darüberhinaus ist die Verwendung von Raney-Nickel in der betrieblichen Praxis nur unter aufwendigen Sicherheitsvorkehrungen möglich.

Es wird weiterhin angegeben, dass das Reduktionsprodukt unbeständig sei und an der Luft rasch oxidiert werde, so dass das Amin nicht isoliert, sondern in Form des mit Ammoniumchlorid angefallenen Gemisches unmittelbar zur Diazotierung eingesetzt wurde.

Es wurde nun gefunden, dass das Monohydrat des 5-Aminobenzimidazolon-(2) an der Luft nur schwach oxidationsempfindlich ist, was seine technische Weiterverarbeitung auf Farbmittel sehr erleichtert. Das Hydrat lässt sich ausserdem in einer grobkristallinen, gut filtrierbaren Form herstellen, was seine Isolierung und die Abtrennung von Verunreinigungen vereinfacht.

Gegenstand der Erfindung ist somit das 5-Amino-benzimidazol-2-on-Monohydrat sowie ein Verfahren zu seiner Herstellung und seine Verwendung als Farbenvorprodukt.

Die erfindungsgemässe Herstellung des 5-Amino-benzimidazol-2-on-Monohydrats erfolgt durch katalytische Reduktion von 5-Nitro-benzimidazolon-(2) und ist dadurch gekennzeichnet, dass man die Reduktion als katalytische Hydrierung im wässrigen Medium und in Gegenwart einer Base durchführt, das 5-Amino-benzimidazolon-(2) durch Säurezusatz aus der Lösung seines Salzes freisetzt und das auskristallisierte Hydrat isoliert.

Als Basen kommen solche Verbindungen in Betracht, die mit dem 5-Nitro- und dem 5-Amino-benzimidazolon-(2) zur Salzbildung befähigt sind, vorzugsweise anorganische Basen, wie wässriges Ammoniak, Alkalihydroxide oder Salze starker Basen mit schwachen Säuren, die in wässriger Lösung durch Hydrolyse basisch reagieren, wie Natriumcarbonat, Dinatriumphosphat oder Borax.

Insbesondere werden jedoch Alkalihydroxide, vor allem Natriumhydroxid, eingesetzt.

Als Säuren kommen grundsätzlich alle sauren Verbindungen in Betracht, die in der Lage sind, aus dem erhaltenen Aminobenzimidazolon-Salz das Amin wieder freizusetzen. In Frage kommen somit beispielsweise Kohlendioxid, bevorzugt jedoch starke Mineralsäuren, wie Schwefelsäure, Salpetersäure und insbesondere Salzsäure.

Besonders bevorzugte Ausgestaltungen der Erfindung werden im folgenden näher erläutert:

Die katalytische Hydrierung erfolgt unter üblichen Bedingungen. Da im alkalischen Medium gearbeitet wird, können ausser Edelmetallkatalysatoren wie Platin oder Palladium auch die billigen Nickelkatalysatoren Verwendung finden. Da das Produkt in Lösung gehen und bleiben soll, hängt die zweckmässige Temperatur von der Menge des Wassers und der Art und Menge der zugesetzten Base ab.

Bevorzugt wird in einem Temperaturbereich von 50 bis 200 °C, insbesondere 80 bis 150 °C gearbeitet. Der Wasserstoffdruck liegt bevorzugt im Bereich von etwa 5 bis 150 bar, insbesondere von 20 bis 100 bar.

Der Säurezusatz erfolgt vorzugsweise nach der Abtrennung des Katalysators, vor allem dann, wenn ein säureempfindlicher Hydrierkatalysator wie Nickel eingesetzt wird.

Die Menge an Base beträgt im allgemeinen ein Äquivalent, bezogen auf das 5-Nitro-benzimidazolon, wobei bevorzugt so verfahren wird, dass die katalytische Reduktion in Gegenwart von etwa $^1/_2$ Äquivalent der Base durchgeführt wird und erst nach der Reduktion das gebildete 5-Aminobenzimidazolon-(2) durch Zusatz eines weiteren halben Äquivalents Base vollständig in das Salz übergeführt wird. Man kann aber auch so vorgehen, dass anstelle des zweiten halben Äquivalents Base soviel Wasser zugesetzt wird, dass das bereits gebildete 5-Amino-benzimidazolon-Salz und das noch vorhandene freie 5-Amino-benzimidazolon-(2) vollständig in Lösung gehen.

Der Zusatz der Säure erfolgt zweckmässig bei einer Temperatur, bei der das 5-Amino-benzimidazolon-(2) gelöst vorliegt, also vorzugsweise bei erhöhter Temperatur, insbesondere bei etwa 60–100 °C. Die Säure wird zweckmässig so bemessen, dass das Reaktionsgemisch neutral reagiert. Ein Überschuss an Säure bringt im allgemeinen keine Vorteile mit sich und belastet nur das Abwasser.

Zweckmässig lässt man nach der Neutralisation das Reaktionsgemisch langsam abkühlen, wobei das Hydrat in grobkristalliner, leicht filtrierbarer Form erhalten wird.

Sofern das Hydrat isoliert und getrocknet werden soll, wird eine Trocknungstemperatur von 60 °C zweckmässig nicht überschritten.

Ein besonders zweckmässiges Herstellungsverfahren besteht somit aus den folgenden Schritten: Eine wässrige Suspension von 5-Nitro-benzimidazolon-(2) wird in einem Hydrierautoklav bei etwa 80 °C mit einem Nickelkontakt und $\frac{1}{2}$ Äquivalent 33 gew.-%iger Natronlauge versetzt. Dann erfolgt die Hydrierung unter üblichen Bedingungen bis zur Beendigung der Wasserstoffaufnahme. Die noch heisse Reduktionsmischung wird mit einem weiteren $\frac{1}{2}$ Äquivalent 33 gew.-%-iger Natronlauge versetzt und kurz nachgerührt. Man filtriert die Reaktionsmischung bei etwa 70–100 °C, bevorzugt 85 °C, in etwa 1 Äquivalent warme, 30 gew.-%ige Salzsäure. Sofern erforderlich, wird der pH-Wert auf 6,5–7,0 eingestellt und die Mischung langsam abgekühlt. Das 5-Amino-benzimidazol-2-on-Monohydrat kristallisiert in Form grober Nadeln aus. Es wird abfiltriert und bei 60 °C im Stickstoffstrom getrocknet. Das Produkt ist an der Luft nur schwach oxidationsempfindlich.

In den folgenden Beispielen wird die Erfindung näher erläutert. Teile und Prozentangaben beziehen sich auf das Gewicht.

### Beispiel 1

896 Teile 5-Nitro-benzimidazolon-(2) werden im Hydrierautoklaven mit 1800 Teilen Wasser bei 80 °C angerührt, mit 7,5 Teilen Nickelkatalysator (z.B. auf Kieselgur) und 300 Teilen 33%iger Natronlauge versetzt und bei 135 °C unter 40 bar Wasserstoffdruck hydriert. Nach Beendigung der Wasserstoffaufnahme wird abgekühlt und bei 90 °C mit 300 Teilen 33%iger Natronlauge versetzt. Man filtriert heiss vom Katalysator in 600 Teile warme 30%ige Salzsäure. Beim Abkühlen der Lösung kristallisiert das 5-Amino-benzimidazol-2-on-Monohydrat in groben, nadelförmigen, gut filtrierbaren Kristallen aus. Das Kristallisat wird bei ca. 20 °C abfiltriert, mit Eiswasser gewaschen und bei 50 °C unter Stickstoff getrocknet. Man erhält 795 Teile 5-Amino-benzimidazol-2-on-Monohydrat in Form grob-nadelförmiger Kristalle.

Analyse für $C_7H_7N_3O \cdot 1H_2O$ (Molgewicht 167,2):

| | C | H | N | O |
|---|---|---|---|---|
| gef.: | 49,6 | 5,4 | 24,9 | 18,2% |
| theor.: | 50,29 | 5,43 | 25,13 | 19,14% |

% $H_2O$ nach K. Fischer:

| | |
|---|---|
| gef.: | 11,1% |
| theor.: | 10,8% |

Differentialthermoanalyse: endothermer Peak bei 110 °C. Das Produkt verliert beim Erhitzen auf 120 °C im Vakuum 1 Mol Wasser und geht in 5-Amino-benzimidazolon-(2) über.

### Beispiel 2

Beispiel 2 wird wie Beispiel 1 mit dem Unterschied ausgeführt, dass vor der kat. Reduktion 600 Teile 33%iger Natronlauge bei 95 °C zugesetzt werden. Dafür entfällt die Zugabe von Natronlauge nach der Reduktion.

Ausbeute und Qualität des 5-Amino-benzimidazolon-(2)-Hydrats entsprechen den Angaben von Beispiel 1.

### Beispiel 3

Die Ausführung von Beispiel 3 entspricht dem Beispiel 1, jedoch mit dem Unterschied, dass die nachträgliche Zugabe von 300 Teilen 33%iger Natronlauge entfällt und stattdessen zur vollständigen Lösung des 5-Amino-benzimidazolons 4000 Teile Wassers zugesetzt werden.

Ausbeute und Qualität des 5-Amino-benzimidazolon-(2)-Hydrats entsprechen den Angaben von Beispiel 1.

### Beispiel 4

Die Ausführung erfolgt wie in Beispiel 1, jedoch mit dem Unterschied, dass die zur Fällung bzw. Kristallisation des Produkts führende Neutralisation mit Salzsäure bei 25 °C erfolgt.

Die Suspension des gebildeten 5-Amino-benzimidazolon-(2)-Hydrats lässt sich wesentlich schwerer filtrieren als das Produkt nach Beispiel 1. Das Schüttgewicht beträgt 400–500 g/l, während das Schüttgewicht des Kristallisats von Beispiel 1 600–800 g/l beträgt.

### Patentansprüche

1. 5-Amino-benzimidazol-2-on-Monohydrat.

2. Verfahren zur Herstellung von 5-Amino-benzimidazol-2-on-Monohydrat, dadurch gekennzeichnet, dass man 5-Nitro-benzimidazolon-(2) in wässrigem Medium und in Gegenwart einer zur Satzbildung mit dem 5-Nitro-benzimidazolon-(2) befähigten Base katalytisch hydriert, das 5-Amino-benzimidazolon-(2) durch Säurezusatz aus der Lösung seines Salzes freisetzt und das auskristallisierte Hydrat isoliert.

3. Verfahren nach Anspruch 2, dadurch gekennzeichnet, dass die Base ein Alkalihydroxid ist.

4. Verfahren nach Anspruch 2 und 3, dadurch gekennzeichnet, dass die Säure Salzsäure ist.

5. Verfahren nach Anspruch 2 bis 4, dadurch gekennzeichnet, dass der Säurezusatz nach Abtrennung des Hydrierkatalysators erfolgt.

6. Verfahren nach Anspruch 2 bis 5, dadurch gekennzeichnet, dass der Säurezusatz bei einer Temperatur von 60–100 °C erfolgt.

7. Verfahren nach Anspruch 2 bis 6, dadurch gekennzeichnet, dass die Reaktionsmischung nach Säurezusatz zur Isolierung des Hydrats abgekühlt wird.

8. Verwendung von 5-Amino-benzimidazol-2-on-Monohydrat als Farbenvorprodukt.

### Revendications

1. Monohydrate d'amino-5-benzimidazolone-2.

2. Procédé de péparation du monohydrate d'amino-5-benzimidazolone-2, procédé caractérisé en ce qu'on hydrogène catalytiquement la nitro-5-benzimidazolone-2 en milieu aqueux et en présence d'une base capable de former un sel avec la nitro-5 benzimidazolone-2, on libère l'amino-5-benzimidazolone-2 de la solution de son sel par addition d'un acide et on isole l'hydrate qui a cristallisé.

3. Procédé selon la revendication 2, caractérisé en ce que la base est un hydroxyde de métal alcalin.

4. Procédé selon l'une des revendications 2 et 3, caractérisé en ce que l'acide est l'acide chlorhydrique.

5. Procédé selon l'une quelconque des revendications 2 à 4, caractérisé en ce qu'on ajoute l'acide après avoir séparé le catalyseur d'hydrogénation.

6. Procédé selon l'une quelconque des revendications 2 à 5, caractérisé en ce qu'on ajoute l'acide à une température de 60 à 100 °C.

7. Procédé selon l'une quelconque des revendications 2 à 6, caractérisé en ce qu'on refroidit le mélange réactionnel, après l'addition d'acide, pour isoler l'hydrate.

8. Application du monohydrate d'amino-5-benzimidazolone-2 comme précurseur de colorants.

**Claims**

1. 5-Amino-benzimidazol-2-one monohydrate.

2. A process for preparing 5-amino-benzimidazol-2-one monohydrate which comprises catalytic hydrogenation of 5-nitro-benzimidazolone-(2) in an aqueous medium containing a base capable of forming a salt with 5-nitro-benzimidazolone-(2), setting free the 5-amino-benzimidazolone-(2) from its salt solution by acidification, and isolation of the crystallized 5-amino-benzimidazol-2-one hydrate.

3. A process as claimed in claim 2, wherein the base is an alkali metal hydroxide.

4. A process as claimed in claim 2 and 3, wherein the acidification is performed by means of hydrochloric acid.

5. A process as claimed in claim 2 to 4, wherein the acidification is performed after removing the hydrogenation catalyst.

6. A process as claimed in claim 2 to 5, wherein the acidification is performed at a temperature of 60 to 100 °C.

7. A process as claimed in claim 2 to 6, wherein the reaction mixture after acidification is cooled for isolating the hydrate.

8. Use of 5-Amino-benzimidazol-2-one monohydrate as a preliminary product for dyestuffs.